Europäisches Patentamt

European Patent Office

Office européen des brevets

(11) Publication number: **0 225 777 B1**

# EUROPEAN PATENT SPECIFICATION

(45) Date of publication of patent specification: **20.05.92**

(51) Int. Cl.⁵: **C12N  15/00**, A01N 63/00, C12N 5/00, C12N 7/00

(21) Application number: **86309368.8**

(22) Date of filing: **02.12.86**

(54) **Insecticides and viruses.**

(30) Priority: **02.12.85 JP 271154/85**

(43) Date of publication of application:
**16.06.87 Bulletin  87/25**

(45) Publication of the grant of the patent:
**20.05.92 Bulletin  92/21**

(84) Designated Contracting States:
**DE FR GB IT NL**

(56) References cited:

**COMPTES RENDUS ACAD. SCID, vol. 290, 25th February 1980, pages 579-582, Paris, FR; G. CROIZIER et al.: "Virologie - Sélection de types viraux dans les infections doubles à Baculovirus chez les larves de Lépidoptère"**

**JOURNAL OF VIROLOGY, vol. 34, no. 3, June 1980, pages 693-703; M.D. SUMMERS et al.: "Physical maps of autographa californica and Rachiplusia ou nuclear polyhedrosis virus recombinants"**

(73) Proprietor: **Maeda, Susumu Toridai Shukusha RC2-103 110, Nishi 4-chome Koyamacho Tottori-shi Tottori-ken(JP)**

(72) Inventor: **Maeda, Susumu Toridai Shukusha RC2-103 110, Nishi 4-chome Koyamacho Tottori-shi Tottori-ken(JP)**

(74) Representative: **Holdcroft, James Gerald, Dr. et al Graham Watt & Co., Riverhead Sevenoaks, Kent TN13 2BN(GB)**

Rank Xerox (UK) Business Services
(3.08/2.19/2.0)

## Description

This invention relates to a so-called viral insecticide which is effective in destroying or controlling injurious insects by using microorganisms pathogenic to them, and to a preparation process thereof.

Viral insecticides adapted to perform bacterial destruction or control of injurious insects by using microorganisms pathogenic to them are attracting interests in recent years, because the microbial insecticides do not give chemical injury to plants and animals. Among such microbial insecticides, certain virus preparations containing nuclear polyhedrosis virus (hereinafter abbreviated as "NPV") as active ingredients have already been put on the market in the United States. The recent state of art of bacterial insecticides was reported for example by Ayusawa, Fujiyoshi, et. al. in "Hakko Kogaku", 51, 351-365 (1973). Kamizumi and Katagiri reported separately the utilization of bacterial insecticides in "Hakko Kogaku", 51, 365-374 (1973).

Conventional virus preparations however encounter difficulties in their mass production and have hence poor utility in view of their production costs, since they are each prepared by feeding an artificial feed, which has been added with NPV of a specific host insect, to larvae of the insect so as to infect the NPV to the larvae, allowing both larvae and NPV to multiply as a result of the feeding of the artificial feed, collecting remains of diseased insects, grinding them, and then forming the resultant powder as a preparation.

Since the host range of insect viruses are generally specified to limited species of insects, there is another problem that it is impossible to obtain any single virus preparation capable of showing insecticidal effects against a wide variety of insects.

It is therefore essential to overcome the above-mentioned problems in order to use a virus preparation actually as an agricultural chemical for destroying or controlling injurious insects.

With the foregoing in view, the present invention has as one object the provision of a virus preparation capable of destroying or controlling two species of injurious insects at the same time by forming a recombinant virus (a hybrid of two viruses of different species) using established insect cell lines to obtain a virus capable of showing different host specificity.

Another object of this invention is to provide a process for the preparation of the recombinant virus, the active ingredient of the above-described virus preparation, which process can also be carried out by a mass production method using a silkworm, Bombyx mori, by a method similar to those conducted in sericulture.

In one aspect of this invention, there is thus provided an insecticide comprising, as an active ingredient, a recombinant virus obtained by infecting a mixture of both strains of two nuclear polyhedrosis viruses, the host insects of which are different from each other, to two types of cells successively, one of said two types of cells permitting multiplication of only one of the two nuclear polyhedrosis viruses and the other allowing multiplication of the other nuclear polyhedrosis virus only, the said host insects being a silkworm, Bombyx mori, and a tobacco cutworm, Spondoptera litura.

In another aspect of this invention, there is also provided a process for the preparation of an insecticide, which comprises infecting a mixture of both strains of two nuclear polyhedrosis viruses, the host insects of which are different from each other, to a first type of cells in which only one of the viruses is allowed to multiply, culturing the thus-infected first type of cells, collecting the supernatant of the resultant culture mixture, infecting the viruses in the supernatant to a second type of cells in which the other virus is solely allowed to multiply, culturing the thus-infected second type of cells, collecting the supernatant of the resultant culture mixture, infecting the viruses in the supernatant again to a monolayer supply of the first type of cells, and then cloning the resultant mixture by a plaque assay to obtain a recombinant virus consisting of a genetically uniform virus strain, and then forming the recombinant virus, either as is or after infecting same to silkworm larvae and multiplying same therein, as an active ingredient into a preparation.

In order to facilitate the understanding of the insecticide and its preparation process to both of which the present invention is directed, a description will hereinafter be made of one example in which an NPV using both of the silkworm, Bombyx mori, and a tobacco cutworm, Spodoptera litura as host insects is employed. Namely, a recombinant virus is formed in the following procedure by using the NPV T3 strain of the silkworm and the NPV OT 102 strain of the tobacco cutworm as two species of NPVs and BmN cells derived from the silkworm and CLS79 cells (ATCC cell line CRL 9373) or SF21AE cells (ATCC cell line 9374) derived from a related species of the tobacco cutworm as cells derived from the host cells.

First of all, a mixture of the NPV T3 strain of silkworm and NPV OT 102 strain of the tobacco cutworm is infected to the CLS 79 cells or SF21AE cells. This infection may preferably be carried out at a rate of 5 virus particles per cell (m.o.i. 5). The thus-infected cells are then cultured for 2 - 3 days. The cell fluid of the culture is collected and thereafter infected to the BmN cells (ATCC cell line CRL 8910) derived from the silkworm. After the infection, the cells are inoculated on a culture medium and then cultured for 3 days.

After the culture, the supernatant of the culture mixture is collected and infected again to a monolayer of CLS79 cells or SF21AE cells.

The thus-infected cells contain two types of species of viruses together, one being capable of forming polyhedral inclusion bodies and the other being practically incapable of forming any inclusion body. A recombinant virus which does not form any polyhedral inclusion body and has genetic uniformity can be obtained by cloning a virus of the latter type selectively by a plaque assay.

When the above-formed recombinant virus was infected to BmN cells derived from the silkworm, it underwent multiplication there. It has hence been confirmed that the strain of the recombinant virus can multiply in both CLS79 cells or SF21AE cells derived from the related species of the tobacco cutworm and BmN cells derived from the silkworm.

For mass production of the recombinant virus as an insecticide formed in the above manner, larvae of the silkworm, which has been improved as a domestic animal in sericulture for several thousand years, can be used at a low cost

Incidentally, the recombinant virus of this invention was multiplied in BmN or SF21AE cells and viral DNA was extracted from the resultant viral particles. DNA fragments which had been obtained by treating the DNA with restriction enzymes were investigated by agarose-gel electrophoresis and DNA hybridization. As a result, the virus was found to contain a part of the DNAs of both of the two species of the NPV T3 (a strain of the silkworm) and the NPV OT 102 (a strain of the tobacco cutworm) and hence to be a recombinant virus of both viruses.

By combining two species of NPVs, the host insects of which are different from each other, using the above-mentioned genetic technique, it is possible to form a recombinant virus capable of infecting both of the host insects and multiplying therein as described above. The present invention has hence opened the door to provide in a large volume a virus preparation at a low cost, which is useful in destroying or controlling a wide variety of injurious insects, by forming a recombinant virus from two species of NPVs the host insects of which are different from each other, infecting the recombinant virus to silkworm larvae and allowing it to multiply there, and then forming the thus-multiplied recombinant virus into a preparation.

For example, a virus preparation which can destroy or control both of a white butterfly, Pieris rapae, an insect injurious to cabbage, and a diamond back moth, Plutella xylostella can be prepared by forming a recombinant virus from a virus infectious to Pieris rapae and another virus infectious to Plutella xylostella and then using the recombinant virus as an active ingredient.

Since the present invention can form a recombinant virus practically incapable of forming any syncytium as described above, the present invention has a merit that an insecticidally-active ingredient free of persistent toxicity can be provided by infecting the recombinant virus to silkworm larvae and then culturing it on a large scale. Namely, the recombinant virus substantially incapable of forming any polyhedral inclusion body is embedded in inclusion bodies of NPV of the silkworm when the recombinant virus is infected to and multiplied in silkworm larvae together with NPV of the silkworm. Therefore, the resulting insecticide remains stable for the period until the insecticide is sprayed in the field. When it is sprayed, the recombinant virus is released from its embedded state and can no longer survive for any long period of time, thereby solving the problem of persistent toxicity.

The preparation of the insecticide of this invention may be effected preferably in the following manner. After confirming the safety of the recombinant virus formed in the above-described manner to small animals, an extender such as bentonite, kaolin or talc the particle size of which is generally about 200 mesh or so is added. One or more substances capable of enhancing the insecticidal activities may also be added in small amounts as needed. The resultant mixture is then formed into a preparation.

Although the recombinant virus, the active ingredient of the insecticide of this invention, was not formed by techniques of genetic engineering like a so-called recombinant DNA experiment, the resulting viruses are considered to be safer insecticides. It is expected that the above-mentioned recombinant viruses can be made by a genetic technique such as that routinely used in recombinant DNA experiments.

The present invention and its advantageous effects will hereinafter be described specifically by the following examples.

Example 1:

Established cells of CLS79 cells (of a species related to the tobacco cutworm, Spodoptera litura) were cultured as a monolayer at 27°C in a petri dish of 60 mm across until the number of cells reached $1.5 \times 10^6$ cells/petri dish. After the culture, the culture medium was removed from the petri dish. A mixture of the BmNPV NPV T3 (ATCC Virus Deposit VR 2163) strain and the SℓNPV NPV OT 102 strain (ATCC Virus Deposit VR 2162) was infected in the below-described manner to the cells left over in the petri dish. First of all, about 0.1 mℓ of a virus solution was prepared to contain about 5 PFUs (plaque forming units) per cell (about 5 m.o.i.). The virus solution was added to each cell in the petri dish and the resultant mixture was allowed to stand for 1 hour to infect the virus to the cells. During this period of time, the petri dish was tiled from time to time.

After completion of the above infecting treatment, the virus solution was removed from the petri dish and the cells still remaining in the petri dish were washed three times with IPL-41 liquid culture medium so as to remove unadsorbed virus from the petri dish.

Thereafter, 4.5 mℓ of a liquid culture medium containing 10% fetal calf serum was added to the cells in the petri dish. The cells were cultured at 27°C. The supernatant of the culture mixture was sampled respectively 24 hours and 40 hours later. The thus-obtained supernatant samples were each diluted to 10 - 1000 times, whereby various virus solutions having different virus concentrations were obtained. By using each of the thus-diluted solutions separately, the virus in the diluted solution was infected to BmN established cells derived from the silkworm, followed by culture of the thus-infected cells for 2 - 3 days. After completion of the culture, the supernatant of each culture mixture was collected and the virus contained in the supernatant was again infected to CLS79 cells Cloning was then conducted by a plaque assay to form a recombinant virus.

Each of the thus-obtained clone strains was infected separately to both BmN cells and CLS79 cells. A clone strain capable of undergoing multiplication in both cells was selected and employed as an insecticidally active ingredient.

By the way, the thus-obtained virus can be infected to silkworm larvae for its mass multiplication.

Formation of the recombinant virus into a preparation:

The recombinant virus formed in the above-described manner was orally infected to third-instar silkworms and at the end of fifth instar, those perished with one or more diseases were collected. A buffer was added to the thus-collected remains and the resultant mixture was ground, followed by centrifugation at 3000 r.p.m for 10 minutes. The resultant precipitate was separated and collected. The thus-obtained precipitate was formed into powder by a method known per se in the art, thereby obtaining an insecticide.

Insecticidal Test:

The above-obtained preparation was added at a dilution of 1/1000 to an artificial feed for tobacco cutworms, Spodoptera mori. After feeding the artificial feed, the tobacco cutworms were allowed to grow to investigate their infection by NPV. It was confirmed that 100% of the tobacco cutworms were infected and perished in two weeks.

Example 2:

A recombinant virus was formed in the same manner as in Example 1 except that established cells of SF21AE cells were used instead of the established cells of CLS79 cells and the cell concentration in a petri dish was adjusted to $1.0 \times 10^6$ cells/petri dish.

The thus-obtained recombinant virus was able to undergo multiplication in both SF21AE cells and BmN cells.

The recombinant virus was formed into a preparation in the same manner as in Example 1, thereby obtaining an insecticide.

Using the above-obtained insecticide, an insecticidal test was conducted under the same conditions as in Example 1. Similar to Example 1, it was confirmed that 100% of the tobacco cutworms were infected and perished in two weeks.

**Claims**

1. An insecticide comprising, as an active ingredient, a recombinant virus obtained by infecting a mixture of both strains of two nuclear polyhedrosis viruses, the host insects of which are different from each other, to two types of cells successively, one of said two types of cells permitting multiplication of only one of the two nuclear polyhedrosis viruses and the other allowing multiplication of the other nuclear polyhedrosis virus only, the said host insects being a silkworm, Bombyx mori, and a tobacco cutworm, Spondoptera litura.

2. The insecticide as claimed in Claim 1, which comprises, as an active ingredient, a recombinant virus obtained by infecting a mixture of both strains of two nuclear polyhedrosis viruses, the host insects of which are different from each other, to a first type of cells in which only one of the viruses is allowed to multiply, culturing the thus-infected first type of cells, collecting the supernatant of the resultant culture mixture, infecting the viruses in the supernatant to a second type of cells in which the other virus is solely allowed to multiply, and culturing the thus-infected second type of cells, the host insects being a silkworm, Bombyx mori, and a tobacco cutworm, Spondoptera litura.

3.  A process for the preparation of an insecticide, which comprises infecting a mixture of both strains of two nuclear polyhedrosis viruses, the host insects of which are different from each other, to a first type of cells in which only one of the viruses is allowed to multiply, culturing the thus-infected first type of cells, collecting the supernatant of the resultant culture mixture, infecting the viruses in the supernatant to a second type of cells in which the other virus is solely allowed to multiply, culturing the thus-infected second type of cells, collecting the supernatant of the resultant culture mixture, infecting the viruses in the supernatant again to a fresh supply of the first type of cells, and then cloning the resultant mixture by a plaque assay to obtain a recombinant virus consisting of a genetically uniform virus strain, and then forming the recombinant virus, either as is or after infecting same to silkworm larvae and multiplying same therein, as an active ingredient into a preparation.

4.  The process as claimed in Claim 4, wherein the host insects are a silkworm, Bombyx mori, and a tobacco cutworm, Spondoptera litura.

5.  The process as claimed in Claim 4, wherein the first and second species of viruses are CLS79 cells (ATCC cell line CRL 9373) or SF21AE cells (ATCC cell line CRL 9374) derived from a related species of a tobacco cutworm, Spondoptera litura and BmN cells derived from the silkworm, Bombyx mori, respectively.

6.  A method of destroying or controlling insects using an insecticide according to Claim or Claim 2.

**Revendications**

1.  Insecticide comprenant, en tant que principe actif, un virus recombinant obtenu en contaminant avec un mélange des deux souches de deux virus polyédrose nucléaire, dont les insectes-hôtes sont différents entre eux, deux types de cellules successivement, l'un de ces deux types de cellules permettant la multiplication de seulement un des deux virus de polyédrose nucléaire et l'autre type permettant la multiplication de l'autre virus de polyédrose nucléaire uniquement, les insectes-hôtes étant un ver à soie, Bombyx mori et une chenille du tabac Spondoptera litura.

2.  Insecticide selon la revendication 1, qui comprend en tant que principe actif, un virus recombinant obtenu en contaminant avec un mélange des deux souches de deux virus de polyédrose nucléaire dont les insectes-hôtes sont différents entre eux, un premier type de cellules dans lequel un type seulement des virus est autorisé à se multiplier, à mettre en culture le premier type ainsi infecté de cellules, à recueillir le surnageant du mélange de culture résultant, en se servant des virus du surnageant pour contaminer un second type de cellules dans lequel l'autre virus est uniquement autorisé à se multiplier et à mettre en culture le second type de cellules ainsi infectées, les insectes-hôtes étant un ver à soie Bombyx mori et une chenille du tabac Spondoptera litura.

3.  Procédé pour la préparation d'un insecticide qui comprend la contamination avec un mélange des deux souches de deux virus de polyédrose nucléaire, dont les insectes-hôtes sont différents l'un de l'autre, un premier type de cellules dans lequel seul un type des virus est autorisé à se multiplier, à mettre en culture le premier type ainsi infecté de cellules, a recueillir le surnageant du mélange de culture résultant, à contaminer avec les virus dans le surnageant un second type de cellules dans lequel l'autre virus est uniquement autorisé à se multiplier, à mettre en culture le second type de cellules ainsi infecté, à recueillir le surnageant du mélange de culture résultant, à contaminer de nouveau avec les virus du surnageant une charge fraîche du premier type de cellules et ensuite à cloner le mélange résultant par un essai à plaque pour obtenir un virus recombinant constitué par une souche virale génétiquement uniforme et ensuite à former le virus recombinant soit tel quel, soit après avoir contaminé avec celui-ci des larves de ver à soie et à les y faire multiplier, sous forme de principe actif dans une préparation.

4.  Procédé selon la revendication 4, dans lequel les insectes-hôtes sont le ver à soie Bombyx mori et une chenille du tabac Spondoptera litura.

5.  Procédé selon la revendication 4, dans lequel la première et seconde espèces de virus sont les cellules CLS79 (lignée cellulaire ATCC CRL 9373) ou les cellules SF21AE (lignée cellulaire ATCC CRL 9374) dérivées d'une espèce apparentée d'une chenille du tabac Spondoptera litura et des cellules BmN dérivées du ver à soie, Bombyx mori respectivement.

6. Procédé destiné à détruire ou maîtriser les insectes en utilisant un insecticide selon la revendication 1 ou la revendication 2.

**Patentansprüche**

1. Insektizid umfassend als einen aktiven Bestandteil, einen rekombinanten Virus, erhalten durch aufeinanderfolgendes Infizieren von zwei Zelltypen mit einem Gemisch aus zwei Stämmen von Kern-Polyeder-Viren, deren Wirts-Insekten verschieden voneinander sind, wobei einer der beiden Zelltypen die Vermehrung von nur einem der beiden Kern-Polyeder-Viren erlaubt, und der andere die Vermehrung nur des anderen Kern-Polyeder-Virus erlaubt, wobei die Wirts-Insekten, ein Seidenspinner, Bombyx mori, und eine Tabak-Motte, Spondoptera litura, sind.

2. Insektizid nach Anspruch 1 umfassend als einen aktiven Bestandteil, einen rekombinanten Virus, erhalten durch Infektion eines ersten Zell-Typs, in dem nur einer der Viren sich vermehren kann, mit einem Gemisch aus zwei Stämmen von Kern-Polyeder-Viren, deren Wirts-Insekten verschieden voneinander sind, das Kultivieren des so infizierten ersten Zell-Typs, das Sammeln des Überstands des resultierenden Kulturgemisches, das Infizieren eines zweiten Zell-Typs, in dem nur der andere Virus sich vermehren kann, mit den Viren aus dem Überstand, und das Kultivieren des so infizierten zweiten Zell-Typs, wobei die Wirts-Insekten ein Seidenspinner, Bombyx mori, und eine Tabak-Motte, Spondoptera litura, sind.

3. Verfahren zur Herstellung eines Insektizids umfassend das Infizieren eines ersten Zell-Typs, in dem sich nur einer der Viren vermehren kann, mit einem Gemisch aus zwei Stämmen von Kern-Polyeder-Viren, deren Wirts-Insekten verschieden voneinander sind, das Kultivieren des so infizierten ersten Zell-Typs, das Sammeln des Überstands des resultierenden Kulturgemisches, das Infizieren eines zweiten Zell-Typs, in dem nur der andere Virus sich vermehren kann, mit den Viren aus dem Überstand, das Kultivieren des so infizierten zweiten Zell-Typs, das Sammeln des Überstands des resultierenden Kulturgemisches, das Infizieren eines frischen Vorrats von Zellen des ersten Typs wiederum mit den Viren aus dem Überstand, und dann das Klonieren des resultierenden Gemisches mittels eines Plaque-Assays, um einen rekombinanten Virus zu erhalten, der aus einem genetisch einheitlichen Virusstamm besteht, und dann das Bilden eines rekombinanten Virus, entweder so wie er ist oder nach Infektion von Seidenspinner-Larven mit ihm und Vermehrung des Virus in den Larven als einen aktiven Bestandteil in einer Präparation.

4. Verfahren nach Anspruch 3, bei dem die Wirts-Insekten ein Seidenspinner, Bombyx mori, und eine Tabak-Motte, Spondoptera litura, sind.

5. Verfahren nach Anspruch 3, bei dem die erste und zweite Spezies von Viren CLS79-Zellen (ATCC Zellinie CRL 9373) oder SF21AE-Zellen (ATCC Zellinie CRL 9374), abgeleitet von einer verwandten Spezies einer Tabak-Motte, Spondoptera litura, beziehungsweise BmN-Zellen, abgeleitet von dem Seidenspinner, Bombyx mori, sind.

6. Verfahren zur Vernichtung oder Kontrolle von Insekten unter Verwendung eines Insektizids nach Anspruch 1 oder 2.